# EUROPEAN PATENT APPLICATION

(11) **EP 4 792 804 A1**
(43) Date of publication of application: **19.08.2026**
(21) Application number: 24891786.6
(22) Date of filing: 14.11.2024
(51) Int. Cl.: A61K 9/127, A61K 38/26, A61K 9/00

(54) **LIPOSOME CONTAINING SEMAGLUTIDE OR PHARMACEUTICALLY ACCEPTABLE SALT THEREOF, PHARMACEUTICAL COMPOSITION COMPRISING SAME, AND PREPARATION METHOD THEREFOR**

(30) Priority: 17.11.2023 KR 20230159792
(71) Applicant: Inventage Lab Inc., Gyeonggi-do 13403 (KR)
(72) Inventor: KIM, Ju Hee, Seongnam-si, Gyeonggi-do 13494 (KR); LEE, Slgirim, Seongnam-si, Gyeonggi-do 13494 (KR); CHOE, Hyeongju, Seongnam-si, Gyeonggi-do 13494 (KR)
(74) Representative: BCKIP Part mbB
(86) International application number: PCT/KR2024/018002
(87) International publication number: WO 2025/105840

(57) **Abstract**

The present invention relates to a liposome containing semaglutide or a pharmaceutically acceptable salt thereof, a pharmaceutical composition comprising same, and a method for preparing same. Semaglutide or a pharmaceutically acceptable salt thereof, which usually exhibits low absorption, can be encapsulated within the liposome, thereby enhancing the absorption rate thereof. In addition, the liposome comprising semaglutide or a pharmaceutically acceptable salt thereof can exhibit excellent effects in regulating blood glucose and preventing and treating obesity by increasing bioavailability. Furthermore, the present invention relates to a method for preparing liposomes containing semaglutide or a pharmaceutically acceptable salt thereof that are uniform in size and exhibit an excellent encapsulation rate and excellent stability.

## Description

### Technical Field

The present invention relates to a liposome containing semaglutide or a pharmaceutically acceptable salt thereof, a pharmaceutical composition comprising the same, and a method for producing the same.

### Background Art

Semaglutide is a GLP-1 (glucagon-like peptide 1) receptor agonist that is used as an agent for treatment of type 2 diabetes and obesity and is classified as a long-term agonist.

Semaglutide is used to help regulate blood sugar levels. It is a GLP-1 analog that helps regulate blood sugar levels by promoting insulin secretion, slowing gastric emptying, and reducing appetite. Thus, it is effective in suppressing the rise in blood sugar levels after meals and lowering HbA1c levels.

In addition, semaglutide may also be used for the treatment of obesity. Its effectiveness as a treatment for weight loss has been proven in clinical practice, and it helps reduce appetite and calorie intake, especially in obese patients. Thus, it may be useful for patients who wish to lose weight.

However, semaglutide is mainly used as an injectable formulation. The injectable formulation is a once-daily formulation with improved convenience, but it should be kept refrigerated because its efficacy decreases when exposed to room temperature for a long time. In addition, because patients have to self-inject the injectable formulation, compliance decreases due to fear and aversion, and consequently, the therapeutic effect also decreases. Furthermore, problems arise, such as causing pain to the patient receiving the injection, the risk of secondary infection, and the generation of medical waste.

Meanwhile, oral formulations have excellent ease of administration, but have disadvantages in that they have a very low absorption rate with a bioavailability of 1% and cause side effects such as vomiting, nausea, and diarrhea.

In order to resolve the above-described problems, it is necessary to research and develop an oral formulation with excellent ease of administration that enhances efficacy and bioavailability to the levels of existing injectable formulations.

### [Prior Art Documents]

### [Patent Documents]

(Patent Document 1) KR 10-2272671B1

### DISCLOSURE

### Technical Problem

An object of the present invention is to provide a liposome containing semaglutide or a pharmaceutically acceptable salt thereof, a pharmaceutical composition comprising the same, and a method for producing the same.

Another object of the present invention is to provide a liposome containing semaglutide or a pharmaceutically acceptable salt thereof, which may increase the absorption rate by incorporating semaglutide or the pharmaceutically acceptable salt thereof, which has an inherently low absorption rate, into the liposome.

Still another object of the present invention is to provide a pharmaceutical composition comprising a liposome containing semaglutide or a pharmaceutically acceptable salt thereof, which may increase bioavailability and exhibit excellent effects on the regulation of blood sugar levels and the prevention and treatment of obesity.

Yet another object of the present invention is to provide a method for producing liposomes containing semaglutide or a pharmaceutically acceptable salt thereof, which have a uniform size, excellent encapsulation efficiency, and excellent stability.

### Technical Solution

To achieve the above objects, the present invention may relate to a liposome containing semaglutide or a pharmaceutically acceptable salt thereof, which has an internal space defined by a lipid layer membrane and contains semaglutide or the pharmaceutically acceptable salt thereof encapsulated in the internal space defined by the lipid layer membrane.

In addition, the lipid layer may comprise polyethylene glycol (PEG)- unconjugated phospholipid, polyethylene glycol-conjugated phospholipid, and cholesterol.

In addition, the polyethylene glycol (PEG)-unconjugated phospholipid may be selected from the group consisting of 1,2-dimyristoyl-sn-glycero-3-phosphocholine (DMPC), 1,2-dihexanoyl-sn-glycero-3-phosphocholine, 1,2-diheptanoyl-sn-glycero-3-phosphocholine, 1,2-dioctanoyl-sn-glycero-3-phosphocholine, 1,2-dinonanoyl-sn-glycero-3-phosphocholine, 1,2-didecanoyl-sn-glycero-3-phosphocholine, 1,2-diundecanoyl-sn-glycero-3-phosphocholine, 1,2-dilauroyl-sn-glycero-3-phosphocholine (DLPC), 1,2-ditridecanoyl-sn-glycero-3-phosphocholine, 1,2-dipentadecanoyl-sn-glycero-3-phosphocholine, 1,2-dipalmitoyl-sn-glycero-3-phosphocholine (DPPC), 1,2-diheptadecanoyl-sn-glycero-3-phosphocholine, 1,2-distearoyl-sn-glycero-3-phosphocholine (DSPC), 1,2-distearoyl-sn-glycero-3-phosphorylethanolamine (DSPE), 1,2-dinonadecanoyl-sn-glycero-3-phosphocholine, 1,2-diarachidoyl-sn-glycero-3-phosphocholine, 1,2-dihenarachidoyl-sn-glycero-3-phosphocholine, 1,2-dibehenoyl-sn-glycero-3-phosphocholine, 1,2-ditricosanoyl-sn-glycero-3-phosphocholine, 1,2-dilignoceroyl-sn-glycero-3-phosphocholine, hydrogenated phosphatidylcholine, 1,2-dioleoylsn-glycero-3-phosphocholine (DOPC), L-α-phosphatidylcholine (HSPC), 1-myristoyl-2-stearoyl-sn-glycero-3-phosphocholine (MSPC), 1-palmitoyl-2-oleoyl-sn-glycero-3-phosphocholine (POPC), phosphatidylethanolamine (POPE), 1-myristoyl-2-palmitoyl-sn-glycero-3-phosphocholine (MPPC), and mixtures thereof.

In addition, the polyethylene glycol-conjugated phospholipid may be selected from the group consisting of PEG-conjugated DMPC, PEG-conjugated 1,2-dihexanoyl-sn-glycero-3-phosphocholine, PEG-conjugated 1,2-diheptanoyl-sn-glycero-3-phosphocholine, PEG-conjugated 1,2-dioctanoyl-sn-glycero-3-phosphocholine, PEG-conjugated 1,2-dinonanoyl-sn-glycero-3-phosphocholine, PEG-conjugated 1,2-didecanoyl-sn-glycero-3-phosphocholine, PEG-conjugated 1,2-diundecanoyl-sn-glycero-3-phosphocholine, PEG-conjugated DLPC, PEG-conjugated 1,2-ditridecanoyl-sn-glycero-3-phosphocholine, PEG-conjugated 1,2-dipentadecanoyl-sn-glycero-3-phosphocholine, PEG-conjugated DPPC, PEG-conjugated 1,2-diheptadecanoyl-sn-glycero-3-phosphocholine, PEG-conjugated DSPC, PEG-conjugated DSPE, PEG-conjugated 1,2-dinonadecanoyl-sn-glycero-3-phosphocholine, PEG-conjugated 1,2-diarachidoyl-sn-glycero-3-phosphocholine, PEG-conjugated 1,2-dihenarachidoyl-sn-glycero-3-phosphocholine, PEG-conjugated 1,2-dibehenoyl-sn-glycero-3-phosphocholine, PEG-conjugated 1,2-ditricosanoyl-sn-glycero-3-phosphocholine, PEG-conjugated 1,2-dilignoceroyl-sn-glycero-3-phosphocholine, PEG-conjugated hydrogenated phosphatidylcholine, PEG-conjugated DOPC, PEG-conjugated HSPC, PEG-conjugated MSPC, PEG-conjugated MPPC, and mixtures thereof.

In addition, the lipid layer may comprise polyethylene glycol (PEG)-unconjugated phospholipid, polyethylene glycol-conjugated phospholipids, and cholesterol at a molar ratio of 1:0.5:0.01 to 1:1:0.1.

In addition, the encapsulation efficiency of semaglutide or the pharmaceutically acceptable salt thereof in the liposome may be 80% or more.

In addition, the liposome may have a polydispersity index (PDI) of 0.3 or less.

In addition, the liposome may have a hydrodynamic diameter (HD) of 70 nm to 110 nm.

Another aspect of the present invention for achieving the above objects may relate to a pharmaceutical composition containing semaglutide or a pharmaceutically acceptable salt thereof, which comprises the liposome.

Still another aspect of the present invention for achieving the above objects may relate to a method for producing liposomes containing semaglutide or a pharmaceutically acceptable salt thereof, comprising: a step of preparing a lipid solution by dissolving lipids in an organic solvent; a step of preparing a semaglutide solution by dissolving semaglutide or the pharmaceutically acceptable salt thereof in a solvent; a step of preparing an oil phase solution by mixing the lipid solution and the semaglutide solution; a step of forming liposomes using the oil phase solution and a buffer solution; and a step of removing the organic solvent from the liposomes, followed by sterilization.

In addition, the lipid solution may contain polyethylene glycol (PEG)-unconjugated phospholipid, polyethylene glycol-conjugated phospholipids, and cholesterol at a molar ratio of 1:0.5:0.01 to 1:1:0.1.

In addition, the solvent for preparing the semaglutide solution may be selected from the group consisting of dimethyl sulfoxide (DMSO), methanol, water, IPA, acetone, and mixtures thereof.

In addition, the concentration ratio between semaglutide or the pharmaceutically acceptable salt and the lipids in the oil phase solution may be 1:100 to 1:20.

In addition, the buffer solution may be selected from the group consisting of phosphate buffered saline, HEPES (4-(2-hydroxyethyl)-1-piperazineethanesulfonic acid) buffer, Tris (tris(hydroxymethyl)aminomethane) buffer, and mixtures thereof.

In addition, the step of forming the liposomes may be performed by forming the liposomes using a microfluidic method.

In addition, the microfluidic method may form the liposomes by injecting the oil phase solution and the buffer solution into a microfluidic chip at a flow rate ratio of 1:2 to 1:10.

In addition, the total flow rate (TFR) of the oil phase solution and the buffer solution, which are injected into the microfluidic chip, may be 50 ml/min to 100 ml/min.

In addition, the step of removing the residual solvent may be performed by loading the solution containing the liposomes into a dialysis kit and exchanging the buffer, followed by stirring to remove the residual solvent.

Yet another aspect of the present invention for achieving the above objects may relate to a method for producing liposomes containing semaglutide or a pharmaceutically acceptable salt thereof, comprising: a step of preparing an oil phase solution by dissolving lipids in an organic solvent; a step of preparing an aqueous phase solution by dissolving semaglutide or the pharmaceutically acceptable salt thereof in an aqueous solution; a step of forming liposomes using the oil phase solution and the aqueous phase solution; and a step of removing the organic solvent from the liposomes, followed by sterilization.

In addition, the aqueous phase solution may be prepared by dissolving semaglutide or the pharmaceutically acceptable salt thereof in a buffer solution.

### Advantageous Effects

According to the present invention, it is possible to increase the absorption rate by incorporating semaglutide or a pharmaceutically acceptable salt thereof, which has an inherently low absorption rate, into a liposome.

In addition, it is possible to provide a liposome containing semaglutide or a pharmaceutically acceptable salt thereof, which may increase bioavailability and exhibit excellent effects on the regulation of blood sugar levels and the prevention and treatment of obesity.

In addition, it is possible to provide a method for producing liposomes containing semaglutide or a pharmaceutically acceptable salt thereof, which have a uniform size, excellent encapsulation efficiency, and excellent stability.

### Brief Description of Drawings

FIG. 1 shows a microfluidic chip for producing liposomes according to one embodiment of the present invention.
FIG. 2 shows the results of measuring the polydispersity index (PDI) and hydrodynamic diameter (HD) of liposomes according to one embodiment of the present invention.
FIG. 3 shows the results of measuring the encapsulation efficiency of liposomes according to one embodiment of the present invention.
FIG. 4 shows the results of testing the bioavailability of a liposome formulation containing semaglutide according to one embodiment of the present invention.

### Best Mode

The present invention relates to a liposome containing semaglutide or a pharmaceutically acceptable salt thereof, which has an internal space defined by a lipid layer membrane and contains semaglutide or the pharmaceutically acceptable salt thereof encapsulated in the internal space defined by the lipid layer membrane.

### Mode for Invention

Hereinafter, embodiments of the present invention will be described in detail so that those skilled in the art can easily implement the present invention. However, the present invention may be embodied in various different forms and is not limited to the embodiments described herein.

Semaglutide is a drug developed to treat diabetes. It is mainly used to treat type 2 diabetes and is classified as a glucagon-like peptide-1 (GLP-1) receptor agonist. Semaglutide acts similarly to insulin to regulate blood sugar levels and increase insulin secretion from the pancreas, thereby allowing cells to absorb glucose.

This drug is usually administered as a single injection before a meal and is primarily prescribed for the treatment or prevention of diabetes.

Recently, semaglutide has been increasingly used for the prevention and treatment of obesity, taking advantage of its blood sugar-regulating effects. Obesity is closely associated with cardiovascular diseases such as hypertension and diabetes, and obesity management is an important factor in health.

Obesity is one of the most common nutritional disorders worldwide. According to WHO statistics, approximately 250 million people are currently classified as obese patients, and it is predicted that approximately 300 million people will suffer from obesity in 20 years.

The obesity refers to a state in which body fat is excessively accumulated due to an imbalance in calorie metabolism caused by excessive energy intake or decreased energy consumption. Recently, the incidence of obesity has been rapidly increasing due to the Westernstyle eating habits and lack of exercise of modern people.

In addition, in terms of numerical concepts, obesity is defined as a body mass index (BMI) of 25 or higher, and the BMI measurement method is an obesity measurement method that estimates the amount of body fat through the value obtained by dividing the weight (kg) by the square (m²) of the height.

Semaglutide, a GLP-1 receptor agonist, is considered a promising candidate for the treatment of obesity due to its appetite-suppressing effects in addition to its blood sugar-regulating effects. Clinical studies have shown that semaglutide may have the effect of inducing weight loss, leading to reduced food intake and reduced total energy intake after a meal.

Currently, the most common subcutaneous injections are once-daily or once-weekly injections, which should be kept refrigerated. There is also the inconvenience of the patient having to self-administer the injection, and the efficacy of the drug may decrease when exposed to room temperature for a long time. In addition, Wegovy tablets (an oral formulation of semaglutide) have a very low absorption rate with a bioavailability of about 1% and cause side effects such as vomiting, nausea, and diarrhea.

To resolve the above-described problems, the present invention relates to a liposome containing semaglutide or a pharmaceutically acceptable salt thereof, more specifically, a liposome that has an internal space defined by a lipid layer membrane and contains semaglutide or a pharmaceutically acceptable salt thereof encapsulated in the internal space defined by the lipid layer membrane.

The liposome is a closed vesicle formed of a lipid bilayer using lipids, and has a water phase (inner water phase) within the space of the closed vesicle. The inner water phase contains water, etc. The liposome usually exists in in a state of being dispersed in an aqueous solution (outer water phase) outside the closed vesicle. The liposome may be single lamellar (which is also referred to as monolayer lamellar or unilamellar, and is a structure having a single bilayer membrane) or multilayered lamellar (which is also referred to as multilamellar and is an onionlike structure having multiple bilayer membranes where individual layers are compartmented by water-like layers). In the present invention, the liposome is preferably a single lamellar liposome from the viewpoint of safety and stability in pharmaceutical applications.

The liposome is not particularly limited in terms of form thereof as long as it is a liposome capable of encapsulating a drug. The "encapsulating" means taking a form in which a drug is contained in an inner aqueous phase and a membrane itself with respect to the liposome. For example, the liposome may be a form where a drug is encapsulated within a closed space formed of a membrane, a form where a drug is encapsulated in the membrane itself, or a combination thereof.

The lipid layer membrane of the liposome is characterized by comprising phospholipids and cholesterol. Specifically, the phospholipids may include polyethylene glycol (PEG)-unconjugated phospholipids and polyethylene glycol-conjugated phospholipids.

Examples of the cholesterol may be cholesterol and its derivatives which comprise cyclopentahydrophenanthrene as a basic framework and in which some or all of the carbons of the framework are hydrogenated. For example, cholesterol is preferred. When the average particle diameter of the liposome is reduced to 100 nm or less, the curvature of the lipid membrane may increase. The distortion of membranes arranged in the liposome also increases. It is effective to add cholesterol or the like in order to fill the distortion of membranes due to lipids (membrane-stabilizing effect).

As the cholesterol is contained, the fluidity of the liposome membrane may be lowered by filling the gaps in the liposome membrane, etc.

The polyethylene glycol (PEG)-unconjugated phospholipid may be selected from the group consisting of 1,2-dimyristoyl-sn-glycero-3-phosphocholine (DMPC), 1,2-dihexanoyl-sn-glycero-3-phosphocholine, 1,2-diheptanoyl-sn-glycero-3-phosphocholine, 1,2-dioctanoyl-sn-glycero-3-phosphocholine, 1,2-dinonanoyl-sn-glycero-3-phosphocholine, 1,2-didecanoyl-sn-glycero-3-phosphocholine, 1,2-diundecanoyl-sn-glycero-3-phosphocholine, 1,2-dilauroyl-sn-glycero-3-phosphocholine (DLPC), 1,2-ditridecanoyl-sn-glycero-3-phosphocholine, 1,2-dipentadecanoyl-sn-glycero-3-phosphocholine, 1,2-dipalmitoyl-sn-glycero-3-phosphocholine (DPPC), 1,2-diheptadecanoyl-sn-glycero-3-phosphocholine, 1,2-distearoyl-sn-glycero-3-phosphocholine (DSPC), 1,2-distearoyl-sn-glycero-3-phosphorylethanolamine (DSPE), 1,2-dinonadecanoyl-sn-glycero-3-phosphocholine, 1,2-diarachidoyl-sn-glycero-3-phosphocholine, 1,2-dihenarachidoyl-sn-glycero-3-phosphocholine, 1,2-dibehenoyl-sn-glycero-3-phosphocholine, 1,2-ditricosanoyl-sn-glycero-3-phosphocholine, 1,2-dilignoceroyl-sn-glycero-3-phosphocholine, hydrogenated phosphatidylcholine, 1,2-dioleoylsn-glycero-3-phosphocholine (DOPC), L-α-phosphatidylcholine (HSPC), 1-myristoyl-2-stearoyl-sn-glycero-3-phosphocholine (MSPC), 1-palmitoyl-2-oleoyl-sn-glycero-3-phosphocholine (POPC), phosphatidylethanolamine (POPE), 1-myristoyl-2-palmitoyl-sn-glycero-3-phosphocholine (MPPC), and mixtures thereof, and specifically, may be HSPC and/or POPC, but is not limited to the above examples, and any polyethylene glycol (PEG)-unconjugated phospholipid capable of constituting the lipid layer membrane of the liposome may be used without limitation.

The polyethylene glycol-conjugated phospholipid may be selected from the group consisting of PEG-conjugated DMPC, PEG-conjugated 1,2-dihexanoyl-sn-glycero-3-phosphocholine, PEG-conjugated 1,2-diheptanoyl-sn-glycero-3-phosphocholine, PEG-conjugated 1,2-dioctanoyl-sn-glycero-3-phosphocholine, PEG-conjugated 1,2-dinonanoyl-sn-glycero-3-phosphocholine, PEG-conjugated 1,2-didecanoyl-sn-glycero-3-phosphocholine, PEG-conjugated 1,2-diundecanoyl-sn-glycero-3-phosphocholine, PEG-conjugated DLPC, PEG-conjugated 1,2-ditridecanoyl-sn-glycero-3-phosphocholine, PEG-conjugated 1,2-dipentadecanoyl-sn-glycero-3-phosphocholine, PEG-conjugated DPPC, PEG-conjugated 1,2-diheptadecanoyl-sn-glycero-3-phosphocholine, PEG-conjugated DSPC, PEG-conjugated DSPE, PEG-conjugated 1,2-dinonadecanoyl-sn-glycero-3-phosphocholine, PEG-conjugated 1,2-diarachidoyl-sn-glycero-3-phosphocholine, PEG-conjugated 1,2-dihenarachidoyl-sn-glycero-3-phosphocholine, PEG-conjugated 1,2-dibehenoyl-sn-glycero-3-phosphocholine, PEG-conjugated 1,2-ditricosanoyl-sn-glycero-3-phosphocholine, PEG-conjugated 1,2-dilignoceroyl-sn-glycero-3-phosphocholine, PEG-conjugated hydrogenated phosphatidylcholine, PEG-conjugated DOPC, PEG-conjugated HSPC, PEG-conjugated MSPC, PEG-conjugated MPPC, and mixtures thereof, and specifically, may be PEG-conjugated DSPE, more specifically, DSPE-PEG2000, but is not limited the above examples, and any polyethylene glycol-conjugated phospholipid capable of constituting the lipid layer membrane of the liposome together with the above-described polyethylene glycol-unconjugated phospholipid may be used without limitation.

The lipid layer may comprise polyethylene glycol (PEG)-unconjugated phospholipid, polyethylene glycol-conjugated phospholipids, and cholesterol at a molar ratio of 1:0.5:0.01 to 1:2:0.1, 1:0.6:0.02 to 1:1.9:0.09, 1:0.7:0.03 to 1:1.8:0.08, 1:0.75:0.04 to 1:1.7:0.075, or 1:0.8:0.05 to 1:1.7:0.075. Within the above ranges, liposomes containing semaglutide or a pharmaceutically acceptable salt thereof may be formed, which have a uniform size, excellent encapsulation efficiency, and excellent stability.

The encapsulation efficiency of semaglutide or a pharmaceutically acceptable salt thereof in the liposome may be 80% or more, 80% to 99%, 81% to 98%, 81% to 97%, 81% to 96%, or 81% to 95%. As described above, the liposome of the present invention may be characterized by having excellent encapsulation efficiency of semaglutide or a pharmaceutically acceptable salt thereof.

The liposome may have a polydispersity index (PDI) of 0.3 or less, 0.29 or less, 0.28 or less, 0.27 or less, 0.26 or less, 0.25 or less, 0.24 or less, 0.23 or less, or 0.22 or less.

The hydrodynamic diameter (HD) of the liposome may be 70 nm to 110 nm, 71 nm to 109 nm, 72 nm to 108 nm, 73 nm to 107 nm, 74 nm to 106 nm, 75 nm to 105 nm, 76 nm to 104 nm, 77 nm to 103 nm, 78 nm to 102 nm, 79 nm to 101 nm, or 80 nm to 100 nm.

In addition to the above-described components, the liposome of the present invention may contain a hydrophilic polymer or the like for improving retention in the blood, fatty acid or diacetylphosphate as a membrane structure stabilizer, and α-tocopherol or the like as an antioxidant. In the present invention, it is preferable not to use additives such as dispersion aids that are not approved for use in intravenous injection in pharmaceutical applications, for example, surfactants.

A method for producing liposomes containing semaglutide or a pharmaceutically acceptable salt thereof according to another embodiment of the present invention may comprise: a step of preparing a lipid solution by dissolving lipids in an organic solvent; a step of preparing a semaglutide solution by dissolving semaglutide or the pharmaceutically acceptable salt thereof in a solvent; a step of preparing an oil phase solution by mixing the lipid solution and the semaglutide solution; a step of forming liposomes using the oil phase solution and a buffer solution; and a step of removing the organic solvent from the liposomes, followed by sterilization.

The lipid solution may be prepared by dissolving polyethylene glycol (PEG)-unconjugated phospholipid, polyethylene glycol-conjugated phospholipid, and cholesterol in an organic solvent.

The organic solvent is not particularly limited, but, for example, a water-soluble organic solvent that is optionally mixed with water may be used. Examples of the organic solvent include alcohols such as methanol, ethanol, n-propanol, isopropanol, n-butanol, isobutanol, and t-butanol; glycols such as glycerin, ethylene glycol, and propylene glycol; polyalkylene glycols such as polyethylene glycol; dimethyl sulfoxide (DMSO), acetone, etc. Among these organic solvents, an alcohol is preferred. The alcohol is preferably at least one selected from among ethanol, methanol, 2-propanol, and t-butanol, more preferably at least one selected from among ethanol, 2-propanol, and t-butanol, more preferably ethanol.

The total lipid concentration of the lipid solution may be 5 mg/ml to 150 mg/ml, 5 mg/ml to 140 mg/ml, 5 mg/ml to 130 mg/ml, 5 mg/ml to 120 mg/ml, 5 mg/ml to 110 mg/ml, 5 mg/ml to 100 mg/ml, 5 mg/ml to 90 mg/ml, 5 mg/ml to 80 mg/ml, 5 mg/ml to 70 mg/ml, 5 mg/ml to 60 mg/ml, or 5 mg/ml to 50 mg/ml.

The lipid solution may contain polyethylene glycol (PEG)-unconjugated phospholipid, polyethylene glycol-conjugated phospholipid, and cholesterol at a molar ratio of 1:0.5:0.01 to 1:2:0.1, 1:0.6:0.02 to 1:1.9:0.09, 1:0.7:0.03 to 1:1.8:0.08, 1:0.75:0.04 to 1:1.7:0.075, or 1:0.8:0.05 to 1:1.7:0.075. Within the above range, liposomes comprising semaglutide or a pharmaceutically acceptable salt thereof may be formed, which have a uniform size, excellent encapsulation efficiency, and excellent stability.

Semaglutide or the pharmaceutically acceptable salt thereof is water-soluble and typically exhibits low solubility in organic solvents. Thus, to prepare a semaglutide solution, a solvent selected from the group consisting of dimethyl sulfoxide (DMSO), methanol, water, IPA, acetone, and mixtures thereof may be used. Among these solvents, DMSO is preferred.

The lipid solution and the semaglutide solution may be mixed together to prepare an oil phase solution. The concentration ratio between semaglutide or a pharmaceutically acceptable salt thereof and the lipids in the oil phase solution may be 1:100 to 1:20, 1:100 to 1:30, 1:100 to 1:40, 1:100 to 1:50, 1:90 to 1:50, 1:80 to 1:50, or 1:70 to 1:50. The concentration ratio may affect the encapsulation efficiency of semaglutide or the pharmaceutically acceptable salt thereof in the liposome. Accordingly, excellent encapsulation efficiency characteristics may be achieved when the concentration ratio between semaglutide or the pharmaceutically acceptable salt thereof and total lipids in an oil phase solution is satisfied within the range described above.

The buffer solution may be selected from the group consisting of phosphate-buffered saline, HEPES (4-(2-hydroxyethyl)-1-piperazineethanesulfonic acid) buffer, Tris (tris(hydroxymethyl)aminomethane) buffer, and mixtures thereof.

The method of forming the liposomes using the oil phase solution and the buffer solution may be performed using any known method for forming liposomes.

As an example, an aqueous solution containing lipids may be prepared by mixing an oil phase and a water phase and stirred to form an emulsion. By mixing and stirring the oil phase in which lipids are dissolved in an organic solvent and the water phase to form an emulsion, an oil-in-water (O/W) emulsion is prepared from the oil phase and the water phase. After mixing, liposomes are formed by removing a portion or all of the organic solvent derived from the oil phase by evaporation. Alternatively, a portion or all of the organic solvent in the oil phase evaporates during the stirring and emulsification process, so that liposomes are formed. As a stirring method, sonication or mechanical shearing is used to obtain fine particles. In addition, to make the particle diameter uniform, an extruder process or a microfluidizer process may be performed by passing the particles through a filter with a constant pore diameter. By using an extruder or the like, the multilamellar liposomes formed secondarily may be broken down into unilamellar liposomes. The emulsification process is not limited as long as it is any emulsifying process, but it is preferably an emulsification process using an organic solvent, which is a process of micronizing by applying high shear. High shear is defined as the peripheral speed of the stirring blades of the emulsifying device, and is preferably 5 m/s to 32 m/s, particularly preferably 20 m/s to 30 m/s. If necessary, the organic solvent used in the emulsification process may be evaporated (removed) to form liposomes.

The liquid temperature in the emulsification process when producing liposomes may be adjusted appropriately, but it is preferable to make the liquid temperature when mixing the oil phase and the water phase equal to or higher than the phase transition temperature of the lipids used. For example, when lipids having a phase transition temperature of 35 to 40°C are used, the liquid temperature is preferably 35°C to 70°C.

However, when liposomes are formed by the emulsification process described above, the diameter of the formed liposomes is not uniform, Thus, a process of processing the liposomes to have a uniform diameter using filtration or extrusion is required.

Accordingly, as a method for forming the liposome in the present invention, for example, a microfluidic method may be used to form the liposome.

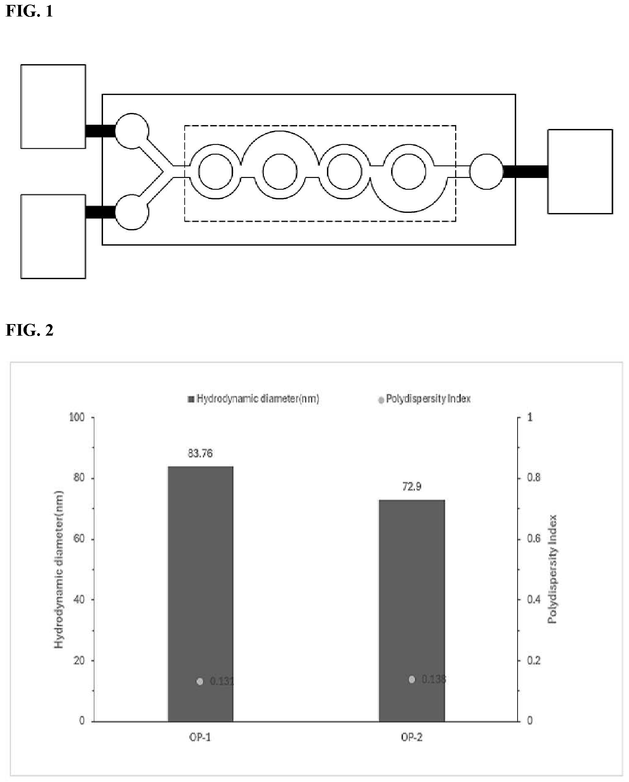

The microfluidic method involves injecting an oil phase solution and a buffer solution into a microfluidic chip as shown in FIG. 1, and allowing the oil phase solution and the buffer solution to flow through the microfluidic chip at a constant flow rate ratio, thereby forming a laminar flow between the oil phase solution and the buffer solution and allowing them to be completely mixed. At this time, liposomes may be formed at the interface between the oil phase solution and the buffer solution.

More specifically, syringes may be connected to the inlet of the microfluidic chip of FIG. 1 by tubing, and an oil phase solution and buffer solution loaded in the syringes may be discharged to a mixer by a syringe pump. At this time, the total flow rate (TFR) may be 50 mL/min to 100 mL/min, 50 mL/min to 90 mL/min, 50 mL/min to 85 mL/min, 50 mL/min to 80 mL/min, 50 mL/min to 75 mL/min, 50 mL/min to 70 mL/min, 55 mL/min to 70 mL/min, or 60 mL/min to 70 mL/min. Within the above range, the Reynolds number (Re) is 2,000, which may correspond to laminar flow that is a fluid state in which ordered layers of fluid particles move.

More specifically, the flow rate ratio between the oil phase solution and the buffer solution, which are discharged to the microfluidic chip, may be 1:2 to 1:10, 1:3 to 1:9, 1:3 to 1:8, 1:3 to 1:7, 1:3 to 1:6, or 1:3 to 1:5. When the flow rate ratio is within the above range while satisfying the total flow rate described above, the Reynolds number is 200, and a laminar flow may be formed, so that liposomes may be formed.

The step of removing the organic solvent may comprise loading the solution containing the liposomes into a dialysis kit, exchanging the buffer, and stirring to remove the organic solvent contained in the solution containing the liposomes. Phosphate-buffered saline buffer may be used as the dialysis fluid. As the organic solvent is removed, the content of the organic solvent in the solution containing the liposomes may be 0.5 vol% or less, preferably 0.05 vol% or less. In addition, the stirring speed may be 500 rpm or less, or 300 rpm.

The solution containing liposomes obtained as described above is preferably subjected to sterile filtration. As a filtration method, a hollow fiber membrane, a reverse osmotic membrane, or a membrane filter may be used to remove unnecessary substances from the solution containing liposomes. In the present invention, the filtration is preferably performed using a filter having a sterilizable pore diameter (preferably a 0.2 µm filtration sterilization filter).

The liposome composition obtained after sterile filtration is preferably aseptically filled for medical applications. Known methods may be applied for aseptic filling. A liposome composition suitable for medical applications may be prepared by aseptically filling the liposome composition in a container.

In addition to the production method described above, it is also possible to prepare an aqueous phase solution by dissolving semaglutide or a pharmaceutically acceptable salt thereof in an aqueous solution, rather than incorporating semaglutide or the pharmaceutically acceptable salt thereof into the oil phase solution, and to use the water phase solution instead of a buffer solution.

The aqueous solution used may be selected from among water (distilled water, water for injection, etc.), saline, various buffer solutions, aqueous solutions of sugars (sucrose, etc.), and mixtures thereof (aqueous solvents).

A pharmaceutical composition containing semaglutide or a pharmaceutically acceptable salt thereof according to another embodiment of the present invention may comprise the liposome.

The liposome composition of the present invention may comprise at least one of pharmaceutically acceptable tonicity agents, stabilizers, antioxidants, and pH adjusting agents depending on the route of administration. That is, the liposome composition of the present invention may be provided as a pharmaceutical composition.

Examples of the tonicity agents include, but are not particularly limited to, inorganic salts such as sodium chloride, potassium chloride, sodium hydrogen phosphate, sodium dihydrogen phosphate, and potassium dihydrogen phosphate; polyols such as glycerol, mannitol, and sorbitol; and sugars such as glucose, fructose, lactose, and sucrose.

Examples of the stabilizers include, but are not particularly limited to, sugars such as glycerol, mannitol, sorbitol, lactose, and sucrose.

Examples of the antioxidants include, but are not particularly limited to, ascorbic acid, uric acid, tocopherol homologues (for example, vitamin E, four tocopherol isomers α, β, γ, and δ), cysteine, and ethylenediaminetetraacetic acid (EDTA). Stabilizers and antioxidants may be respectively used alone or in combination of two or more thereof.

Examples of the pH adjusting agents include sodium hydroxide, citric acid, acetic acid, triethanolamine, sodium hydrogen phosphate, sodium dihydrogen phosphate, and potassium dihydrogen phosphate.

The liposome composition of the present invention may contain a pharmaceutically acceptable organic solvent, collagen, polyvinyl alcohol, polyvinyl pyrrolidone, carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum Arabic, casein, gelatin, agar, diglycerol, propylene glycol, polyethylene glycol, vaseline, paraffin, stearyl alcohol, stearic acid, human serum albumin (HSA), mannitol, sorbitol, lactose, phosphate buffered saline (PBS), sodium chloride, sugars, biodegradable polymer, serum-free medium, or an additive which is acceptable as a pharmaceutical additive.

The container in which the liposome composition of the present invention is filled is not particularly limited, but is preferably made of a material having low oxygen permeability. Examples of the container include a plastic container, a glass container, and a bag made of a laminate film having an aluminum foil, an aluminum-deposited film, an aluminum oxide-deposited film, a silicon oxide-deposited film, polyvinyl alcohol, an ethylene-vinyl alcohol copolymer, polyethylene terephthalate, polyethylene naphthalate, polyvinylidene chloride, or the like as a gas barrier layer. If necessary, light may be shielded by adopting a bag or the like using a colored glass, an aluminum foil, an aluminum-deposited film, or the like.

In the container in which the liposome composition is filled, in order to prevent oxidation by oxygen present in the space of the container, it is preferable to replace the gas in the container space and drug solution with an inert gas such as nitrogen. For example, an injection solution may be bubbled with nitrogen, whereby the filling of the injection solution into the container may be carried out under a nitrogen atmosphere.

The administration route of the pharmaceutical composition of the present invention may be parenteral administration or oral administration. Examples of the parenteral administration include intravenous injection such as intravenous drip, intramuscular injection, intraperitoneal injection, subcutaneous injection, intraocular injection, and intrathecal injection. The administration method may be, for example, administration by a syringe or intravenous drip.

The dosage and frequency of administration of the pharmaceutical composition according to the present invention may be appropriately set depending on the type of drug, the condition of the patient, and the like. The dose of the pharmaceutical composition may be generally set in the range of 0.01 mg/kg/day to 100 mg/kg/day in terms of the amount of drug which is an active ingredient. The dose of the pharmaceutical composition may be set in the range of 2 mg to 10 mg per dose in terms of the amount of drug which is an active ingredient, but it is not limited to these dosages.

The pharmaceutical composition of the present invention, which comprises semaglutide or a pharmaceutically acceptable salt thereof, may be used for purposes such as preventing or treating obesity, lowering blood sugar levels, treating or preventing diabetes, etc., because it comprises semaglutide or the pharmaceutically acceptable salt thereof.

### Preparation Example

The composition of lipids is shown in Table 1 or 2 below, and a lipid solution was prepared by dissolving the lipids shown in Table 1 or 2 below in pure ethanol.

A semaglutide solution was prepared by dissolving semaglutide in dimethyl sulfoxide (DMSO) at a concentration of 3.33 mg/mL.

An oil phase solution was prepared by mixing the lipid solution and the semaglutide solution. The weight ratio between lipids to semaglutide in the oil phase solution was 100:1, 50:1, or 20:1.

10 mM phosphate buffered saline (pH 7.4) was used as an aqueous solution.

A microfluidic chip shown in FIG. 1 was used, and each syringe was connected to the inlet of the microfluidic chip of FIG. 1 by tubing. The oil phase solution and aqueous solution loaded into the syringes were discharged into the microfluidic chip by a syringe pump (Chemyx). At this time, the total flow rate (TFR) was 68 mL/min. At this time, the Reynolds number (Re) was 2,000. In addition, the flow rate ratio (FRR, buffer solution: buffer solution) was 3:1. The oil phase solution and the aqueous phase solution passed through the microfluidic chip, mixed together, and formed liposomes, and the solution containing the formed liposomes was collected in a vial.

The solution containing the liposomes was loaded into a dialysis kit and dialyzed while stirring with phosphate-buffered saline buffer. Residual ethanol and DMSO in the solution containing the liposomes were removed by a buffer exchange and stirring process. The amount of residual ethanol was reduced to 0.02% or less of the total solution.

The solution containing the liposomes was sterilized by passage through a filter (0.2 µm).

**[Table 1]**

| Lipids | Molar ratio | mg/mL |
|---|---|---|
| HSPC | 52 | 18.66 |
| Cholesterol | 45 | 7.96 |
| DSPE-PEG2000 | 3 | 3.82 |

**[Table 2]**

| Lipids | Molar ratio | mg/mL |
|---|---|---|
| POPC | 52 | 18.66 |
| Cholesterol | 45 | 7.96 |
| DSPE-PEG2000 | 3 | 3.82 |

### Experimental Example

### Examination of Conditions Depending on Co-Solvent and Dissolution Conditions

To select a co-solvent for dissolving semaglutide in an oil phase solution, PBS, methanol, and DMSO were used as solvents for preparing the semaglutide solution.

The test conditions are shown in Table 3 below.

**[Table 3]**

| Sema-Liposome | OP-1 | OP-2 | OP-3 |
|---|---|---|---|
| Composition | POPC/Chol/DSPE-PEG2000 | | |
| | Molar ratio (%) 52:45:3 | | |
| API Solubility | API+PBS | API+MeOH | API+DMSO |
| Lipid to API Ratio | 100 | | |
| Total Volume (mL) | 12 | | |
| Total Flow Rate (TFR-mL/min) | 68 | 54 | |
| Flow Rate Ratio (FRR) (Aqueous : Lipid sol) | 5:1 | | |
| Initial Lipid Conc. (in solvent) mg/mL | 45.69 | | |

The results of measuring the polydispersity index (PDI) and hydrodynamic diameter (HD) of each of OP-1 and OP-2 are shown in FIG. 2. Referring to FIG. 2, it was confirmed that liposomes with a uniform diameter were formed.

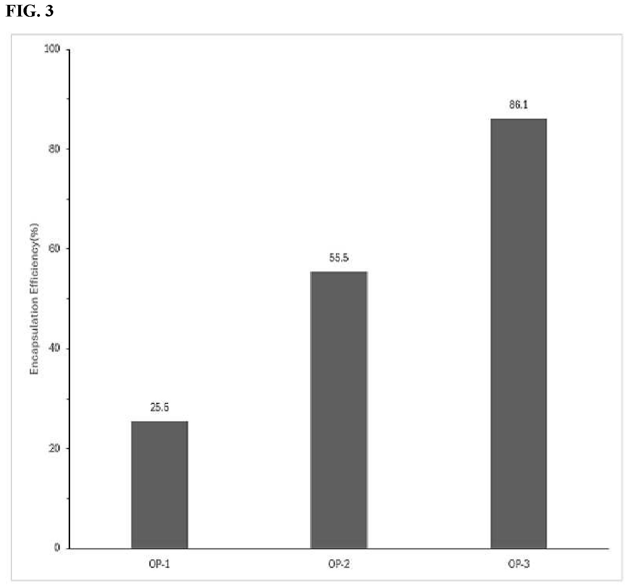

FIG. 3 shows the results of measuring the encapsulation efficiency of each of OP-1 to OP-3. It was confirmed that there was a significant difference in the encapsulation efficiency of semaglutide in liposomes depending on the type of co-solvent. Accordingly, DMSO was finally selected as the co-solvent and subsequent experiments were conducted.

### Comparison of Physicochemical Properties Depending on Flow Rate Ratio (FRR, Buffer Solution: Buffer Solution) Injected into Microfluidic Chip

To produce liposomes, liposomes were produced while changing the flow rate ratio between the oil phase solution and the aqueous phase solution, and their characteristics were compared.

The test conditions are shown in Table 4 below.

**[Table 4]**

| Sema-Liposome | OP-60 | OP-61 | OP-62 |
|---|---|---|---|
| Composition | HSPC/Chol/DSPE-PEG2000 | | |
| | Molar ratio (%) 42:55:3 | | |
| Lipid to API Ratio | 100 | | |
| Total Flow Rate (TFR-mL/min) | 68 | | |
| Flow Rate Ratio (FRR) | 3:1 | 5:1 | 9:1 |

For the produced liposomes, hydrodynamic diameter (HD) and polydispersity index (PDI) were measured by dynamic light scattering (DLS), zeta potential by electrophoretic light scattering (ELS), and encapsulation efficiency (EE, %) by HPLC. The results are shown in Table 5 below.

**[Table 5]**

| Physicochemical properties | OP-60 | OP-61 | OP-62 |
|---|---|---|---|
| Hydrodynamic diameter | 82.62 | 91.02 | 105.5 |
| PDI | 0.162 | 0.155 | 0.172 |
| Zeta Potential | -10.86 | -11.4 | -10.67 |
| EE (%) | 85.84 | 86.77 | 79.29 |

Referring to the above test results, it was confirmed that the diameter increased with changes in the flow rate ratio, and that the encapsulation efficiency was 80% or less at a flow rate ratio of 9:1.

### Comparison of Physicochemical Properties Depending on Molar Ratio between Phospholipids and Cholesterol

Liposomes must not release drugs outside the liposomes during storage outside the body. Therefore, the encapsulation efficiency was evaluated while changing the molar ratio between phospholipids and cholesterol forming the bilayer. At this time, liposomes were produced while changing the molar ratio from 1.10:1 to 1:1.06 or 1:1.3, and their physicochemical properties were compared.

**[Table 6]**

| | | | |
|---|---|---|---|
| Composition | HSPC/Chol/ DSPE-PEG2000 | | |
| Molar ratio (%) | 52:45:3 | 47:50:3 | 42:55:3 |
| Phospholipid:chol | 1.10:1 | 1:1.06 | 1:1.3 |
| Lipid to API Ratio | 100 | | |
| Total Flow Rate (TFR-mL/min) | 68 | | |
| Flow Rate Ratio (FRR) | 3:1 | | |

For the produced liposomes, hydrodynamic diameter (HD) and polydispersity index (PDI) were measured by dynamic light scattering (DLS), zeta potential by electrophoretic light scattering (ELS), and encapsulation efficiency (EE, %) by HPLC. The results are shown in Table 7 below.

**[Table 7]**

| | | | |
|---|---|---|---|
| Molar ratio (%) | 52:45:3 | 47:50:3 | 42:55:3 |
| Hydrodynamic diameter | 91.72 | 83.08 | 81.53 |
| PDI | 0.220 | 0.1942 | 0.1645 |
| Zeta Potential | -10.53 | -9.61 | -12.02 |
| EE (%) | 93.55 | 78.94 | 84.36 |

Referring to the above test results, it was confirmed that the diameter and polydispersity index increased slightly depending on the change in the molar ratio between phospholipids and cholesterol, that the encapsulation efficiency was 90% or higher when the molar ratio between phospholipids and cholesterol was 1.10:1. Thus, the molar ratio between phospholipids and cholesterol was set to 1.10:1 and subsequent experiments were conducted.

### Comparison of Physicochemical Properties Depending on Weight Ratio of Total Lipids to Drug

The ratio of total lipids to drug in liposomes can affect storage stability and duration. Accordingly, liposomes were produced by adjusting the weight ratio between semaglutide and total lipids as shown in Table 8 below, and the hydrodynamic diameter (HD) and polydispersity index (PDI) were measured by dynamic light scattering (DLS), the zeta potential by electrophoretic light scattering (ELS), and the encapsulation efficiency (EE, %) by HPLC. The results are shown in Table 9 below.

**[Table 8]**

| | | | | |
|---|---|---|---|---|
| Composition | HSPC/Chol/ DSPE-PEG2000 | | | |
| | Molar ratio (%) 52:45:3 | | | |
| Lipid:API | 100:1 | 75:1 | 50:1 | 25:1 |
| Total Flow Rate (TFR-mL/min) | 68 | | | |
| Flow Rate Ratio (FRR) | 3:1 | | | |

**[Table 9]**

| | | | | |
|---|---|---|---|---|
| Lipid:API | 100:1 | 75:1 | 50:1 | 25:1 |
| Hydrodynamic diameter | 86.16 | 88.59 | 87.56 | 83.86 |
| PDI | 0.2127 | 0.185 | 0.1898 | 0.1938 |
| Zeta Potential | -10.36 | -9.02 | -9.99 | -10.57 |
| EE (%) | 89.97 | 90.46 | 92.94 | 87.58 |

Referring to the above test results, there was little change in diameter and polydispersity index depending on the change in the total lipid-to-drug ratio. However, when the weight ratio between total lipids and semaglutide was 75:1 and 50:1, the encapsulation efficiency was excellent at 90% or higher.

### Characteristics Depending on Organic Solvent Removal Conditions Used in Liposome Production

Ethanol was used to prepare the total lipid solution, and DMSO was used as the solvent for dissolving the drug. Liposomes were produced and loaded into a dialysis device, and the concentration of the organic solvents (ethanol & DMSO) used was reduced and removed by replacing the external buffer.

The amount of organic solvent used is defined in the ICH Q3 guideline, and ethanol and DMSO, which are solvents used in liposome production, are classified as Class 3 solvents and should not exceed 5,000 ppm or 0.5%.

Accordingly, the amounts of residual organic solvents following the removal of the organic solvents and the resulting drug encapsulation efficiency were measured, and the results are shown in Table 10 below.

**[Table 10]**

| | | | |
|---|---|---|---|
| Composition | POPC/Chol/DSPE-PEG2000 | | |
| Molar ratio (%) | 52:45:3 | | |
| Lipid to API Ratio | 100 | | |
| Total Flow Rate | 54 | | |
| Final EtOH Conc. (%) | 0.013 | 0.033 | 0.011 |
| Final DMSO Conc. (%) | 0.0018 | 0.0046 | 0.0015 |
| EE (%) | 89.97 | 90.46 | 92.94 |

Referring to the above test results, it was confirmed that the liposome encapsulation efficiency changed depending on the residual organic solvent concentrations, and specifically, the encapsulation efficiency increased when the concentrations of the residual organic solvents were reduced.

### Change in Encapsulation Efficiency (%) Depending on Colloidal Stability of Liposome Solution

Since liposomes cause changes in colloidal stability depending on the surface charge or particle size in the dilution solvent, the same volume was obtained from the liposome solution to compare the content, and the total amount of drug and its ratio in the aliquot volume were examined. Table 11 below shows the conditions for liposome production, and as shown in Table 12 below, liposomes 26-1, 26-2, and 26-3 were produced through repeated production, and their characteristics were examined.

**[Table 11]**

| | |
|---|---|
| Composition | POPC/Chol/ DSPE-PEG2000 |
| Molar ratio (%) | 52:45:3 |
| Lipid to API Ratio | 100 |
| Total Flow Rate (TFR-mL/min) | 68 |
| Flow Rate Ratio (FRR) | 5:1 |

**[Table 12]**

| Sample | 26-1 | | 26-2 | | | | 26-3 | | |
|---|---|---|---|---|---|---|---|---|---|
| Total volume (mL) | 13 | | | | | | | | |
| Aliquot volume (mL) | 4, 4 and 5 | | | | | | | | |
| Hydrodynamic diameter | 82.58 | | | 82.49 | | | 86.95 | | |
| PDI | 0.143 | | | 0.162 | | | 0.164 | | |
| Zeta Potential | -9.913 | | | -6.664 | | | -9.266 | | |
| Theoretical content (mg) | 0.223 | | 0.278 | 0.211 | | 0.264 | 0.211 | | 0.264 |
| API Content (mg) | 0.206 | 0.214 | 0.268 | 0.196 | 0.201 | 0.253 | 0.190 | 0.204 | 0.251 |

Referring to the above test results, a content test was performed by aliquoting 4 mL or 5 mL of solution from the liposome solution under the same conditions, and it was confirmed that the difference in the amount of drug in the obtained liposome compared to the theoretical content was 10% or less, indicating the colloidal stability of the liposome in the solution.

### Physicochemical Properties of Liposome Solution under Refrigeration Conditions

Since storage conditions and usage periods are supported by data on the chemical and physical stability of liposomes, evaluation of physical and chemical changes in liposomes during refrigerated storage (temperature: 5±3°C) was performed.

The conditions for producing liposomes are shown in Table 13 below.

**[Table 13]**

| | |
|---|---|
| Composition | HSPC/Chol/ DSPE-PEG2000 |
| Molar ratio (%) | 52:45:3 |
| Lipid to API Ratio | 100 |
| Total Flow Rate (TFR-mL/min) | 68 |
| Flow Rate Ratio (FRR) | 3:1 |

The test results are shown in Table 14 below.

**[Table 14]**

| | | | Weeks | | | | |
|---|---|---|---|---|---|---|---|
| Test item | | | 0 | 1 | 2 | 3 | 4 |
| Appearance | Visual | Milky white particles Suspension | Milky white particles Suspension | Milky white particles Suspension | Milky white particles Suspension | Milky white particles Suspension | Milky white particles Suspension |
| Content | HPLC | 90.0 to 110.0 % | 101.68 | 95.09 | 95.18 | 95.18 | 99.56 |
| DLS | Hydrodynamic diameter | 70.0 to 100.0 nm | 83.01 | 82.94 | 82.52 | 82.12 | 81.57 |
| | PDI | < 0.3 | 0.201 | 0.2084 | 0.1827 | 0.1935 | 0.1874 |
| | Zeta Potential | -20.0 to 0 | -11.62 | -10.64 | -9.309 | -10.06 | -9.541 |
| Result | | | Pass | Pass | Pass | Pass | Pass |

Referring to the above test results, it was confirmed that the liposome solution remained stable for 4 weeks without any physical or chemical changes under refrigerated storage conditions.

### Examination of Whether the Use of Liposomes Improves Bioavailability

To evaluate the bioavailability of a liposomal formulation of semaglutide using Beagle dogs, an experiment was conducted as follows.

### Experimental Animals and Housing Environment

Beagle dogs (male, about 13 to 14 months old) were used as experimental animals. The animals were housed in an environment with a temperature of 23±3°C, relative humidity of 55±15%, ventilation rate of 10 to 20 times/hr, lighting time of 12 hours (lighting at 8:00 AM to 8:00 PM), and illuminance of 150 to 300 Lux.

### Administration and Method of Administration

Oral administration (PO) was performed on groups G1 and G2 groups as follows.

Group G1: Place the control drug, Rybelsus ablet, on the back of the tongue, allow the mouth to close, gently stroke the throat to induce swallowing, check if swallowed, and administer about 10 mL of water using a syringe.

Group G2: Place a hard capsule containing a pH adjusting agent on the back of the tongue, allow the mouth to close, gently stroke the throat to induce swallowing, check if swallowed, and administer about 10 mL of water using a syringe. After 30 minutes, connect a catheter to a syringe containing liposomes produced under the conditions shown Table 13 above, insert the catheter to reach the stomach, inject the test substance, and administer about 10 mL of water.

### Fasting and Water Restriction

Fasting was performed for at least 16 hours before the administration of the test substance, and feed was provided after blood was collected 1 hour after the administration of the test substance. Water intake was restricted starting from 3 hours before the administration of the test substance, and water was supplied after blood was collected 1 hour after administration.

### Blood Collection and Analysis

Blood collection points were a total of 12 points: before administration of the test substance (0), and at 0.25, 0.5, 0.75, 1, 1.5, 2, 3, 4, 6, 12, and 24 hours after administration. At each point, 3 mL of whole blood was collected via jugular vein. From the samples, the plasma drug concentrations were analyzed using LC-MS/MS (Exion QTRAP5500, SCIEX).

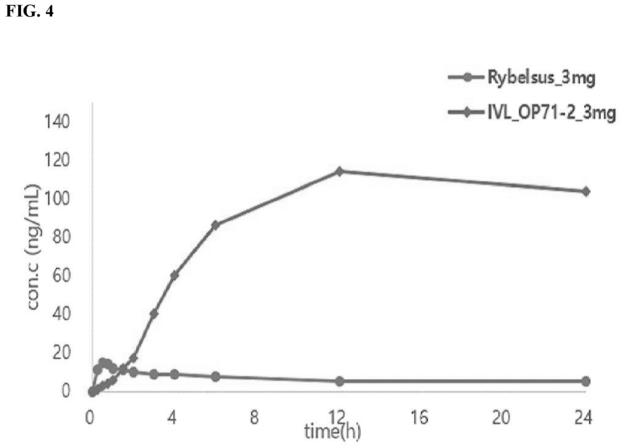

The test results are shown in Table 15 below and FIG. 4.

**[Table 15]**

| Parameter | IVL_OP71-2 | Rybelsus | T/R Ratio |
|---|---|---|---|
| Cₘₐₓ | 114.22 | 15.08 | 7.57 |
| AUC_{0→24h} | 2147.87 | 157.29 | 13.66 |

Referring to the above test results, it was confirmed that, when the liposome containing semaglutide according to the present invention was administered, there was a significant difference in the results of Cₘₐₓ and AUC_{0→24h}. In particular, it was confirmed that there was a difference of about 14 times in AUC_{0→24h}. That is, it was confirmed that the bioavailability increased by about 14 times when administering 3 mg of semaglutide using the liposomal formulation of the present invention compared to when administering 3 mg of semaglutide using the commercially available oral formulation.

Although the preferred embodiments of the present invention have been described in detail above, the scope of the present invention is not limited thereto, and various modifications and improvements made by those skilled in the art using the basic concept of the present invention as defined in the appended claims also fall within the scope of the present invention.

### Industrial Applicability

The present invention relates to a liposome containing semaglutide or a pharmaceutically acceptable salt thereof, a pharmaceutical composition comprising the same, and a method for producing the same.

## Claims

1. A liposome containing semaglutide or a pharmaceutically acceptable salt thereof, which has an internal space defined by a lipid layer membrane and contains semaglutide or the pharmaceutically acceptable salt thereof encapsulated in the internal space defined by the lipid layer membrane.

2. The liposome of claim 1, wherein the lipid layer comprises polyethylene glycol (PEG)-unconjugated phospholipid, polyethylene glycol-conjugated phospholipid, and cholesterol.

3. The liposome of claim 2, wherein the polyethylene glycol (PEG)-unconjugated phospholipid is selected from the group consisting of 1,2-dimyristoyl-sn-glycero-3-phosphocholine (DMPC), 1,2-dihexanoyl-sn-glycero-3-phosphocholine, 1,2-diheptanoyl-sn-glycero-3-phosphocholine, 1,2-dioctanoyl-sn-glycero-3-phosphocholine, 1,2-dinonanoyl-sn-glycero-3-phosphocholine, 1,2-didecanoyl-sn-glycero-3-phosphocholine, 1,2-diundecanoyl-sn-glycero-3-phosphocholine, 1,2-dilauroyl-sn-glycero-3-phosphocholine (DLPC), 1,2-ditridecanoyl-sn-glycero-3-phosphocholine, 1,2-dipentadecanoyl-sn-glycero-3-phosphocholine, 1,2-dipalmitoyl-sn-glycero-3-phosphocholine (DPPC), 1,2-diheptadecanoyl-sn-glycero-3-phosphocholine, 1,2-distearoyl-sn-glycero-3-phosphocholine (DSPC), 1,2-distearoyl-sn-glycero-3-phosphorylethanolamine (DSPE), 1,2-dinonadecanoyl-sn-glycero-3-phosphocholine, 1,2-diarachidoyl-sn-glycero-3-phosphocholine, 1,2-dihenarachidoyl-sn-glycero-3-phosphocholine, 1,2-dibehenoyl-sn-glycero-3-phosphocholine, 1,2-ditricosanoyl-sn-glycero-3-phosphocholine, 1,2-dilignoceroyl-sn-glycero-3-phosphocholine, hydrogenated phosphatidylcholine, 1,2-dioleoylsn-glycero-3-phosphocholine (DOPC), L-α-phosphatidylcholine (HSPC), 1-myristoyl-2-stearoyl-sn-glycero-3-phosphocholine (MSPC), 1-palmitoyl-2-oleoyl-sn-glycero-3-phosphocholine (POPC), phosphatidylethanolamine (POPE), 1-myristoyl-2-palmitoyl-sn-glycero-3-phosphocholine (MPPC), and mixtures thereof.

4. The liposome of claim 2, wherein the polyethylene glycol-conjugated phospholipid is selected from the group consisting of PEG-conjugated DMPC, PEG-conjugated 1,2-dihexanoyl-sn-glycero-3-phosphocholine, PEG-conjugated 1,2-diheptanoyl-sn-glycero-3-phosphocholine, PEG-conjugated 1,2-dioctanoyl-sn-glycero-3-phosphocholine, PEG-conjugated 1,2-dinonanoyl-sn-glycero-3-phosphocholine, PEG-conjugated 1,2-didecanoyl-sn-glycero-3-phosphocholine, PEG-conjugated 1,2-diundecanoyl-sn-glycero-3-phosphocholine, PEG-conjugated DLPC, PEG-conjugated 1,2-ditridecanoyl-sn-glycero-3-phosphocholine, PEG-conjugated 1,2-dipentadecanoyl-sn-glycero-3-phosphocholine, PEG-conjugated DPPC, PEG-conjugated 1,2-diheptadecanoyl-sn-glycero-3-phosphocholine, PEG-conjugated DSPC, PEG-conjugated DSPE, PEG-conjugated 1,2-dinonadecanoyl-sn-glycero-3-phosphocholine, PEG-conjugated 1,2-diarachidoyl-sn-glycero-3-phosphocholine, PEG-conjugated 1,2-dihenarachidoyl-sn-glycero-3-phosphocholine, PEG-conjugated 1,2-dibehenoyl-sn-glycero-3-phosphocholine, PEG-conjugated 1,2-ditricosanoyl-sn-glycero-3-phosphocholine, PEG-conjugated 1,2-dilignoceroyl-sn-glycero-3-phosphocholine, PEG-conjugated hydrogenated phosphatidylcholine, PEG-conjugated DOPC, PEG-conjugated HSPC, PEG-conjugated MSPC, PEG-conjugated MPPC, and mixtures thereof.

5. The liposome of claim 2, wherein the lipid layer comprises the polyethylene glycol (PEG)-unconjugated phospholipid, the polyethylene glycol-conjugated phospholipids, and the cholesterol at a molar ratio of 1:0.5:0.01 to 1:2:0.1.

6. The liposome of claim 1, wherein an encapsulation efficiency of semaglutide or the pharmaceutically acceptable salt thereof in the liposome is 80% or more.

7. The liposome of claim 1, wherein the liposome has a polydispersity index (PDI) of 0.3 or less.

8. The liposome of claim 1, wherein the liposome has a hydrodynamic diameter (HD) of 70 nm to 110 nm.

9. A pharmaceutical composition containing semaglutide or a pharmaceutically acceptable salt thereof, which comprises the liposome according to claims 1 to 8.

10. A method for producing liposomes containing semaglutide or a pharmaceutically acceptable salt thereof, comprising:
a step of preparing a lipid solution by dissolving lipids in an organic solvent;
a step of preparing a semaglutide solution by dissolving semaglutide or the pharmaceutically acceptable salt thereof in a solvent;
a step of preparing an oil phase solution by mixing the lipid solution and the semaglutide solution;
a step of forming liposomes using the oil phase solution and a buffer solution; and
a step of removing the organic solvent from the liposomes, followed by sterilization.

11. The method of claim 10, wherein the lipid solution contains polyethylene glycol (PEG)-unconjugated phospholipid, polyethylene glycol-conjugated phospholipids, and cholesterol at a molar ratio of 1:0.5:0.01 to 1:1:0.1.

12. The method of claim 10, wherein the solvent for preparing the semaglutide solution is selected from the group consisting of dimethyl sulfoxide (DMSO), methanol, water, IPA, acetone, and mixtures thereof.

13. The method of claim 10, wherein a concentration ratio between semaglutide or the pharmaceutically acceptable salt and the lipids in the oil phase solution is 1:100 to 1:20.

14. The method of claim 10, wherein the buffer solution is selected from the group consisting of phosphate buffered saline, HEPES (4-(2-hydroxyethyl)-1-piperazineethanesulfonic acid) buffer, Tris (tris(hydroxymethyl)aminomethane) buffer, and mixtures thereof.

15. The method of claim 10, wherein the step of forming the liposomes is performed by forming the liposomes using a microfluidic method.

16. The method of claim 15, wherein the microfluidic method forms the liposomes by injecting the oil phase solution and the buffer solution into a microfluidic chip at a flow rate ratio of 1:2 to 1:10.

17. The method of claim 16, wherein a total flow rate (TFR) of the oil phase solution and the buffer solution, which are injected into the microfluidic chip, is 50 ml/min to 100 ml/min.

18. The method of claim 10, wherein the step of removing the organic solvent is performed by loading the solution containing the liposomes into a dialysis kit and exchanging the buffer, followed by stirring to remove the organic solvent contained in the solution containing the liposomes.

19. A method for producing liposomes containing semaglutide or a pharmaceutically acceptable salt thereof, comprising:
a step of preparing an oil phase solution by dissolving lipids in an organic solvent;
a step of preparing an aqueous phase solution by dissolving semaglutide or the pharmaceutically acceptable salt thereof in an aqueous solution;
a step of forming liposomes using the oil phase solution and the aqueous phase solution; and
a step of removing the organic solvent from the liposomes, followed by sterilization.

20. The method of claim 19, wherein the aqueous phase solution is prepared by dissolving semaglutide or the pharmaceutically acceptable salt thereof in a buffer solution.
